(19) Europäisches Patentamt
European Patent Office
Office européen des brevets

(11) **EP 3 809 417 A1**

(12) **EUROPEAN PATENT APPLICATION**

(43) Date of publication:
**21.04.2021 Bulletin 2021/16**

(51) Int Cl.:
**G16H 20/17** *(2018.01)*     **A61M 1/16** *(2006.01)*

(21) Application number: **19202905.6**

(22) Date of filing: **14.10.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Gambro Lundia AB**
**220 10 Lund (SE)**

(72) Inventors:
• **Donato, Danilo**
  **35123 Padova (IT)**
• **Storr, Markus**
  **70794 Filderstadt (DE)**

(74) Representative: **Perchenek, Nils**
**Gambro Dialysatoren GmbH**
**Legal and Intellectual Property**
**Holger Crafoord-Strasse 26**
**72379 Hechingen (DE)**

(54) **DETERMINING INTERNAL FILTRATION RATE WITHIN A CAPILLARY DIALYZER**

(57)     The present disclosure relates to a method and a device for determining an internal filtration rate IFR within a capillary hemodialyzer.

Fig. 1

EP 3 809 417 A1

## Description

### Technical Field

The present disclosure relates to a method and a device for determining an internal filtration rate IFR within a capillary hemodialyzer.

### Description of the Related Art

The difficult removal of middle molecules from patients suffering from end-stage kidney disease represents one of the major challenges of hemodialysis. High concentrations of middle molecular weight (MW) solutes, such as $\beta_2$-micro-globulin, myoglobin, complement factor D, and polyclonal free light chains ($\kappa$-FLC and $\lambda$-FLC), have been correlated to critical clinical outcomes in chronic kidney disease patients. In conventional hemodialysis, low MW solutes (e.g. urea and creatinine) are effectively removed by diffusion, whereas the removal of middle MW solutes is generally achieved with the superimposition of convective transport over pure diffusion. The use of more water-permeable high-flux dialyzers and hemodiafiltration to increase convective transport has been shown to enhance the clearance of middle MW solutes, but still leads to unsatisfactory clinical results.

In EP 3 102 312 B1 and EP 3 102 314 B1, a new class of membranes, defined as Medium cut-off (MCO), and hemodialyzers comprising such membranes are disclosed. The particular features of MCO membranes include high MW retention onset and MW cut-off value close to the molecular weight of albumin, allowing for the removal of solutes up to ca. 45 kDa with negligible albumin loss. The use of hemodialyzers equipped with MCO membranes has made it possible to perform a new therapy called expanded hemodialysis (HDx), in which convective and diffusive transport are efficiently combined to increase dialyzer removal capability over a wide range of molecular weights. This is due to the interplay among the larger permeability of the MCO membranes as compared to that of high-flux membranes, and their geometrical structure, which increase convective transport across the hemodialyzer membrane by enhancing internal filtration (i.e. movement of plasma water from the blood compartment towards the dialysate compartment across the porous membrane in the proximal part of the hemodialyzer) and back filtration (i.e. movement of plasma water from the dialysate compartment towards the blood compartment in the distal part of the hemodialyzer) . The compensation of internal filtration (IF) with an adequate amount of back filtration (BF) also permits to avoid a complex set-up and the use of fluid reinfusion, thus overcoming some practical issues of hemodiafiltration.

Methods to experimentally quantify the rate of IF and BF applied to hemodialyzers equipped with MCO membranes have been recently described by A. Lorenzin et al.: "Quantification of internal filtration in hollow fiber hemodialyzers with Medium cut-off membrane", Blood Purif. 46 (2018) 196-204. However, the experimental quantification of IF generally requires complex settings, and it is not cost-effective. Therefore, a simple and effective method for determining internal filtration rate IFR within a hemodialyzer is still lacking.

### Summary

The present disclosure provides a method and a device for determining an internal filtration rate IFR within a capillary hemodialyzer. The method only requires dimensions of the hemodialyzer, dimensions well as physical parameters of the hollow fiber membranes present in the hemodialyzer, and flow rates of blood and dialysate through the hemodialyzer, all of which are readily available.

### Brief Description of the Drawings

Figure 1    shows a schematic perspective view and sectional detail views of a capillary hemodialyzer and fluid flows within the hemodialyzer;

Figure 2    shows a comparison of experimentally determined internal filtration rates and internal filtration rates determined according to the method of the present disclosure for one model of a hemodialyzer;

Figure 3    shows a comparison of experimentally determined internal filtration rates and internal filtration rates determined according to the method of the present disclosure for another model of a hemodialyzer.

## Detailed Description

The present disclosure provides a computer-implemented method for determining an internal filtration rate IFR within a capillary hemodialyzer. The method comprises

> i) acquiring, using an electronic computer processor, data relating to physical properties of the hemodialyzer and of hollow fibers present in the hemodialyzer;
> ii) acquiring, using the processor, a blood flow rate $Q_B$ and a dialysis flow rate $Q_D$ through the hemodialyzer during operation of the hemodialyzer;
> iii) determining, using the processor, the internal filtration rate IFR of the hemodialyzer, based on the data acquired in steps i and ii.

In one embodiment, the method additionally comprises iv) acquiring, using the processor, data on a duration TD of the operation of the hemodialyzer;

> v) determining, using the processor, a total volume $V_{tot}$ of fluid exchanged through the wall of the hollow fiber membranes present in the hemodialyzer during operation of the hemodialyzer, based on the internal filtration rate IFR and the data acquired in step iv.

In a particular embodiment, data relating to physical properties of the hemodialyzer and of hollow fibers present in the hemodialyzer is acquired from a database in operative association with the processor.

In one embodiment of the method, the database comprises data relating to physical properties of a plurality of different hemodialyzers and of hollow fibers present in the hemodialyzers. For each hemodialyzer of the plurality of different hemodialyzers, such data may comprise a diameter $d_H$ of the housing of the hemodialyzer, a number N of the hollow fibers present in the hemodialyzer, an effective length L of the hollow fibers present in the hemodialyzer, a total surface area $A_{tot}$ of the hollow fibers present in the hemodialyzer, an internal diameter $d_B$ of the hollow fibers present in the hemodialyzer, a wall thickness $\delta_M$ of the hollow fibers present in the hemodialyzer, a porosity $\varepsilon_M$ of the hollow fibers present in the hemodialyzer, and an ultrafiltration coefficient $K_{UF}$ of the hollow fibers present in the hemodialyzer.

Additionally, the database may comprise data on a blood viscosity $\mu_B$ and a dialysate viscosity $\mu_D$.

In a further embodiment, data relating to physical properties of the hemodialyzer and of hollow fibers present in the hemodialyzer and/or the blood flow rate $Q_B$ and the dialysis flow rate $Q_D$ through the hemodialyzer during operation of the hemodialyzer is acquired from an input device in operative association with the processor.

The hemodialyzer is perfused with blood at a blood flow rate $Q_B$ and dialysate at a dialysate flow rate $Q_D$ during operation, i.e., when it is used in an external blood circuit to remove toxins from blood. The internal filtration rate IFR and the total volume $V_{tot}$ of fluid exchanged through the wall of the hollow fiber membranes present in the hemodialyzer vary with blood flow rate $Q_B$ and dialysate flow rate $Q_D$ through the hemodialyzer.

In one embodiment, the input device comprises a contactless reader. In a particular embodiment, the contactless reader is an optical reader which acquires data of a barcode or QR code present on a hemodialyzer. In another particular embodiment, the contactless reader is a sensor which acquires data from an RFID or NFC tag present on or in the hemodialyzer.

In a further embodiment, the input device comprises at least one user interface. In a particular embodiment, the user interface comprises a keyboard or a touchscreen. In a further embodiment of the system, the input device comprises a graphical use interface (GUI). In a particular embodiment, the input device comprises a touchscreen of a smartphone. The input device is used for manually entering data relating to physical properties of the hemodialyzer and of hollow fibers present in the hemodialyzer, and/or the blood flow rate $Q_B$ and the dialysis flow rate $Q_D$ through the hemodialyzer during operation of the hemodialyzer.

In another embodiment, the input device comprises an extracorporeal blood treatment apparatus. The extracorporeal blood treatment apparatus controls the parameters of an extracorporeal blood circuit comprising a hemodialyzer during operation, i.e. during the treatment of a patient. When used as an input device for the method of the present disclosure, the extracorporeal blood treatment apparatus provides data relating to physical properties of the hemodialyzer and of hollow fibers present in the hemodialyzer, and/or the blood flow rate $Q_B$ and the dialysis flow rate $Q_D$ through the hemodialyzer during operation of the hemodialyzer. In one embodiment of the method, the processor acquires real-time data of the blood flow rate $Q_B$ and the dialysis flow rate $Q_D$ through a hemodialyzer during operation of the hemodialyzer and, optionally, data on the duration TD of the operation, from the extracorporeal blood treatment apparatus, i.e. during an actual treatment. In another embodiment, a blood flow rate $Q_B$ and a dialysis flow rate $Q_D$ through a hemodialyzer during operation of the hemodialyzer and, optionally, a duration TD of the operation, are manually entered through the input device to simulate a treatment.

In one embodiment of the method, the data relating to physical properties of the hemodialyzer and of hollow fibers present in the hemodialyzer comprise a diameter $d_H$ of the housing of the hemodialyzer, a number N of the hollow fibers present in the hemodialyzer, an effective length L of the hollow fibers present in the hemodialyzer, a total surface area $A_{tot}$ of the hollow fibers present in the hemodialyzer, an internal diameter $d_B$ of the hollow fibers present in the hemodialyzer, a wall thickness $\delta_M$ of the hollow fibers present in the hemodialyzer, a porosity $\varepsilon_M$ of the hollow fibers present in the hemodialyzer, and an ultrafiltration coefficient $K_{UF}$ of the hollow fibers present in the hemodialyzer.

In one embodiment of the method, the internal filtration rate IFR is determined according to

$$IFR = N\pi d_B \int_0^{x_i} J_v(z)\,dz$$

with

N       number of hollow fibers present in the hemodialyzer;
$d_B$      internal diameter of hollow fibers present in the hemodialyzer;
$J_v(z)$    ultrafiltration flux through the membrane wall;
$x_i$       location of inversion point ($J_v(x_i) = 0$).

This equation can be rewritten as

$$IFR = N\pi d_B K \int_0^{x_i} (P_B(z) - P_D(z) - \pi_o)\,dz$$

with

$$K \quad - \frac{K_{UF}}{A_{tot}\,\varepsilon_M},$$

$K_{UF}$ ultrafiltration coefficient of the hollow fibers present in the hemodialyzer,
$A_{tot}$ total membrane area of the hollow fibers present in the hemodialyzer ($A_{tot} = N\pi d_B L$),
$\varepsilon_M$ membrane porosity of the hollow fibers present in the hemodialyzer;

$P_B(z)$    blood pressure;
$P_D(z)$    dialysate pressure;
$\Pi_o$       average oncotic pressure;

In another embodiment of the method, the internal filtration rate IFR is determined according to

$$IFR = N\pi d_B\, K \left\{ (P_{B,out} - P_{D,out} - \pi_0)x_i \right.$$

$$- \frac{128\mu_B}{N\pi d_B^4}\left[ Q_B\left(\frac{x_i^2}{2} - Lx_i\right) - N\pi d_B\left[\frac{v_2 - v_1}{6L}\left(\frac{x_i^4}{4} - L^3 x_i\right) + \frac{v_1}{2}\left(\frac{x_i^3}{3} - L^2 x_i\right)\right]\right]$$

$$\left. + \frac{\mu_D}{2N\pi f_{R_o,R_k}}\left[ Q_D\frac{x_i^2}{2} - N\pi d_B\left[\frac{v_2 - v_1}{2L}\left(\frac{x_i^4}{12} - \frac{L^2}{2}x_i^2\right) + v_1\left(\frac{x_i^3}{6} - \frac{L}{2}x_i^2\right)\right]\right]\right\}$$

with

N   number of hollow fibers present in the hemodialyzer;
$d_B$   internal diameter of the hollow fibers present in the hemodialyzer;

$$\mathbb{K} \qquad - \frac{K_{UF}}{A_{tot}\,\varepsilon_M},$$

$K_{UF}$ ultrafiltration coefficient of the hollow fibers present in the hemodialyzer,
$A_{tot}$ total membrane area of the hollow fibers present in the hemodialyzer ($A_{tot} = N\pi d_B L$),
$\varepsilon_M$ membrane porosity of the hollow fibers present in the hemodialyzer;

$P_{B,out}$   blood pressure at hemodialyzer exit;
$P_{D,out}$   dialysate pressure at hemodialyzer exit;
$\Pi_o$   average oncotic pressure;
$x_i$   location of inversion point ($J_v(x_i) = 0$);
$\mu_B$   blood viscosity;
$Q_B$   blood flow rate;
L   effective length of hollow fibers present in the hemodialyzer;
$v_1$   $J_v(0)$;
$v_2$   $J_v(L)$;
$\mu_D$   dialysate viscosity;
$Q_D$   dialysate flow rate;

$$f_{R_o,R_k} \quad \frac{R_k^4 - R_o^4}{16} + \frac{R_k^2}{2}\left(\frac{R_k^2}{2}\ln\left(\frac{R_o}{R_k}\right) + \frac{R_k^2 - R_o^2}{4}\right) - \frac{R_o^2}{8}\left(R_k^2 - R_o^2\right),$$

$$R_o = \frac{d_B}{2} + \delta_M,$$

$$R_k = R_o + \delta_\varepsilon,$$

$d_B$   internal diameter of hollow fibers present in the hemodialyzer,
$\delta_M$   wall thickness of the hollow fibers present in the hemodialyzer,
$\delta_\varepsilon$   half the distance between adjacent hollow fibers present in the hemodialyzer.

In a further embodiment of the method, some of the parameters in the above equation for IFR are calculated according to the following equations:

1.

$$f_{R_o,R_k} = \frac{R_k^4 - R_o^4}{16} + \frac{R_k^2}{2}\left(\frac{R_k^2}{2}\ln\left(\frac{R_o}{R_k}\right) + \frac{R_k^2 - R_o^2}{4}\right) - \frac{R_o^2}{8}\left(R_k^2 - R_o^2\right)$$

2.

$$K = -\frac{K_{UF}}{A_{tot}\,\varepsilon_M}$$

3.

$$E_1 = \frac{\mu_D d_B L^3}{16 f_{R_o,R_k}} - \frac{80 \mu_B L^3}{3 d_B^3}$$

4.

$$E_2 = \frac{5\mu_D d_B L^3}{48 f_{R_o,R_k}} - \frac{16\mu_B L^3}{d_B^3}$$

5.

$$R_1 = 1 + \frac{128}{3} K \frac{\mu_B L^2}{d_B^3}$$

6.

$$R_2 = 1 - K \frac{\mu_D d_B L^2}{6 f_{R_o,R_k}} + \frac{16}{9} K^2 \frac{\mu_B \mu_D L^4}{R_1 f_{R_o,R_k} d_B^2}$$

7.

$$F_1 = \left(P_{B,out} - \pi_0\right)L + 64 \frac{\mu_B L^2 Q_B}{N\pi d_B^4} + \frac{\mu_D L^2 Q_D}{4N\pi f_{R_o,R_k}}$$

8.

$$F_2 = E_2 - \frac{64}{3} \frac{E_1 \mu_B L^2 K}{R_1 d_B^3}$$

9.

$$B_1 = P_{B,out} - P_{D,out} - \pi_o + \frac{128\mu_B Q_B L}{N\pi d_B^4}$$

10.

$$B_2 = P_{B,out} - \pi_o + \frac{128\mu_B Q_B L}{N\pi d_B^4}$$

11.

$$D_1 = P_{B,out} - P_{D,out} - \pi_o + \frac{\mu_D Q_D L}{2N\pi f_{R_o,R_k}}$$

12.

$$D_2 = P_{B,out} - \pi_o + \frac{\mu_D Q_D L}{2N\pi f_{R_o,R_k}}$$

13.

$$G_1 = F_1 + \frac{E_1}{R_1}KB_2 + \frac{F_2}{R_2}KD_2 + \frac{F_2}{R_2}K^2\frac{\mu_D d_B L^2}{12 f_{R_O,R_k}}B_2$$

14.

$$G_2 = L + \frac{E_1}{R_1}K + \frac{F_2}{R_2}K + \frac{F_2}{R_2}K^2\frac{\mu_D d_B L^2}{12 R_1 f_{R_O,R_k}}$$

15.

$$P_{D,out} = \frac{1}{G_2}\left(G_1 - \frac{UF}{N\pi d_B K}\right)$$

16.

$$v_1 = J_v(0) = K\frac{1}{R_1}\left(B_1 - \frac{64\mu_B L^2}{3d_B^3}v_2\right)$$

17.

$$v_2 = J_v(L) = \frac{K}{R_2}\left(D_1 + K\frac{\mu_D d_B L^2 B_1}{12 R_1 f_{R_O,R_k}}\right)$$

18.

$$x_i = \frac{v_1}{v_1 - v_2}$$

with

| | |
|---|---|
| $N$ | number of hollow fibers present in the hemodialyzer; |
| $L$ | effective length of the hollow fibers present in the hemodialyzer; |
| $d_B$ | internal diameter of the hollow fibers present in the hemodialyzer; |
| $A_{tot}$ | total membrane area of the hollow fibers present in the hemodialyzer ($A_{tot} = N\pi d_B L$); |
| $UF$ | net ultrafiltration flow rate; |
| $K_{UF}$ | UF coefficient; |
| $Q_B$ | blood flow rate; |
| $Q_D$ | dialysate flow rate; |
| $P_{B,out}$ | blood pressure at dialyzer exit; |
| $P_{D,out}$ | dialysate pressure at dialyzer exit; |
| $\pi_o$ | average oncotic pressure; |
| $\mu_B$ | blood viscosity; |
| $\mu_D$ | dialysate viscosity; |
| $\varepsilon_M$ | membrane porosity; |
| $\delta_M$ | membrane thickness. |

In one embodiment of the method, the total volume $V_{tot}$ of fluid exchanged through the wall of the hollow fiber membranes present in the hemodialyzer over a time period TD of operation of the hemodialyzer is determined according to

$$V_{tot} = \int\limits_{0}^{TD} IFR(t)dt$$

The present disclosure also provides a system comprising

a) a database comprising data relating to physical properties of a plurality of capillary hemodialyzers and of hollow fibers present therein; and/or
b) an input device configured for providing data relating to physical properties of a capillary hemodialyzer and of hollow fibers present in the hemodialyzer and/or for providing a blood flow rate and a dialysis flow rate through the hemodialyzer during operation of the hemodialyzer;
c) an output device configured for output of data received from a computer processor in operative association with the output device;
d) a computer processor programmed for communication with the database and/or the input device, and for communication with the output device, the processor programmed for

a. acquiring data from the database and/or the input device,
b. determining an internal flow rate IFR within a capillary hemodialyzer, based on the acquired data,
c. optionally, determining a total volume $V_{tot}$ of fluid exchanged through the wall of the hollow fiber membranes present in the hemodialyzer during operation of the hemodialyzer;
d. transmitting the determined internal flow rate IFR, and, optionally, the total volume $V_{tot}$ of fluid exchanged to the output device.

In one embodiment of the system, the input device, the output device, and the processor are contained in a portable device. In one embodiment, the portable device is a portable computer, for instance, a laptop, a tablet computer, or a PDA. In a further embodiment, the portable device is a mobile communication device, for instance, a smartphone.
In one embodiment of the system, the output device is a display device. Examples of suitable display devices include monitors, computer displays, and touchscreens. In a particular embodiment, the display device is a touchscreen of a smartphone.
In one embodiment of the system, the database is present in a computer memory in operative association with the computer processor. In a further embodiment, the computer memory is contained in a portable device comprising the input device, the output device, and the processor. In another embodiment, the database is present in a remote computer memory, a network drive, or a cloud memory accessible via the internet.
The present disclosure also provides a computer program for instructing a computer processor to perform the method of

a. acquiring data from a database and/or an input device in operative association with the processor;
b. determining an internal filtration rate IFR within a capillary hemodialyzer, based on the acquired data;
c. optionally, determining a total volume $V_{tot}$ of fluid exchanged through the wall of the hollow fiber membranes present in the hemodialyzer during operation of the hemodialyzer;
d. transmitting the determined internal flow rate IFR, and, optionally, the total volume $V_{tot}$ of fluid exchanged to an output device in operative association with the processor.

In one embodiment, the computer program takes the form of a software application ("app") that can be installed and run on a smartphone.
The present disclosure also provides a non-transitory computer-readable medium comprising the computer program.
It will be understood that the features mentioned above and those described hereinafter can be used not only in the combination specified but also in other combinations or on their own, without departing from the scope of the present invention.
The method of the present disclosure will now be further described in the following examples and referring to the attached drawings.

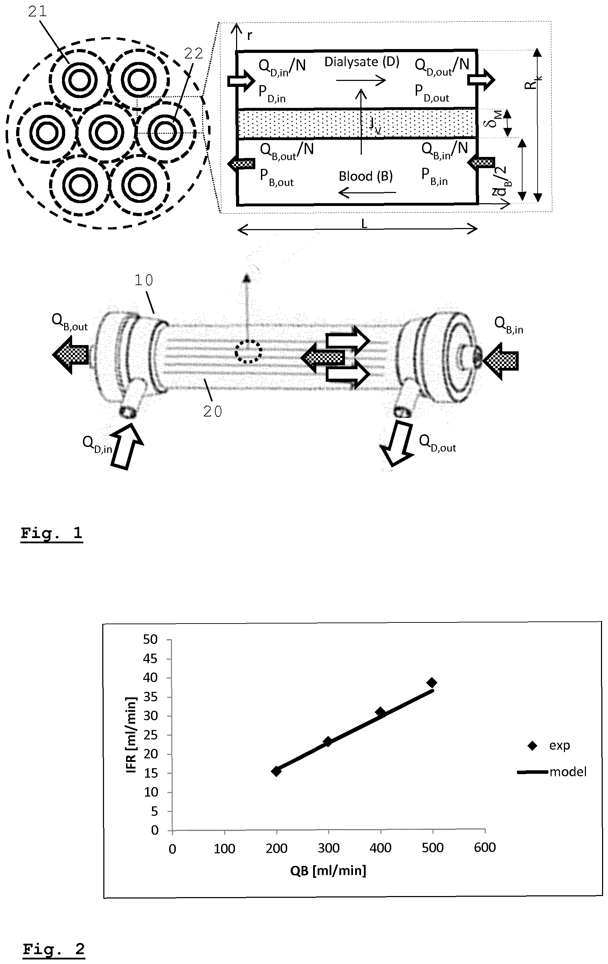

Figure 1 shows a schematic perspective view and sectional detail views of a capillary hemodialyzer 10 and fluid flows within the hemodialyzer 10. The hemodialyzer 10 comprises a bundle 20 of hollow fiber membranes 21. In the top left corner of Fig. 1, a detail of a cross-section of the bundle 20 is shown with several hollow fiber membranes 21, each having a membrane wall 22. Blood flow and dialysate flow through the hemodialyzer 10 are indicated by arrows.

Blood enters the hemodialyzer 10 through a blood inlet located on a header of the hemodialyzer 10 at a blood flow rate $Q_{B,in}$, flows through the lumen of the hollow fiber membranes 21 of the bundle 20, and leaves the hemodialyzer 10 at a blood flow rate $Q_{B,out}$ through a blood outlet located on another header of the hemodialyzer 10. Dialysate enters the hemodialyzer 10 through a dialysate inlet positioned near one end of the hemodialyzer 10 at a dialysate flow rate $Q_{D,in}$, flows through the space outside the hollow fiber membranes 21 of the bundle 20, and leaves the hemodialyzer 10 at a dialysate flow rate $Q_{D,out}$ through a dialysate outlet positioned near the opposite end of the hemodialyzer 10. In the top right corner of Fig. 1, a detail of a longitudinal section of one of hollow fiber membranes 21 is shown. The effective length of the hollow fiber membrane 21 is L, the internal radius of the hollow fiber membrane 21 is $d_B/2$, the thickness of the membrane wall 22 is $\delta_M$, the distance between the longitudinal axis of one hollow fiber membrane 21 and the longitudinal axis of an adjacent hollow fiber membrane 21 is $2*R_k$, with $R_k=R_o+\delta_\varepsilon$, or $R_k=d_B/2+\delta_M+\delta_\varepsilon$, $R_o$ being half the outside diameter of the hollow fiber membrane 21, and $\delta_\varepsilon$ being half the distance between two adjacent hollow fiber membranes 21. Blood flows in the lumen of the hollow fiber membrane 21, dialysate flows on the outside of the hollow fiber membrane 21, and ultrafiltration flux $J_v$ permeates the membrane wall 22.

**Example 1**

Internal filtration rates within a hemodialyzer (Polyflux® 210H, Gambro Dialysatoren GmbH, 72379 Hechingen, Germany) were determined according to one embodiment of the method of the present disclosure, and compared to internal flow rates determined experimentally (from D. Schneditz et al.: "Internal filtration, filtration fraction, and blood flow resistance in high- and low-flux dialyzers", Clin. Hemorheol. Microcirc. 58 (2014) 455-469).
The following physical properties of the hemodialyzer were used for the determination:

- hollow fiber inner diameter $d_B$ = 215 $\mu$m

- Membrane wall thickness $\delta_M$ = 50 $\mu$m

- Membrane porosity $\varepsilon_M$ = 0.7

- Number of fibers N = 11,640

- Effective length L = 270 mm

- Ultrafiltration coefficient $K_{UF}$ = 85 ml/(h*mm Hg)

- Half distance between adjacent fibers $\delta_\varepsilon$ = 68.2 $\mu$m

- Hemodialyzer housing inner diameter $d_H$ = 48.7 mm

Values for blood viscosity of $\mu_B$ = 5.2 mPas and dialysate viscosity of $\mu_D$ = 0.96 mPas were used.
Internal filtration rate IFR was determined for a dialysis flow rate $Q_D$ of 500 ml/min, and blood flow rates $Q_B$ of 200 ml/min, 300 ml/min, 400 ml/min, and 500 ml/min, respectively.
The values for the following constant parameters were obtained as described above.

- K = 0.119 $\mu$m$^2$s/kg

- $R_1$ = 1.19

- $R_2$ = 1.01

- $E_1$ = -285 MPas

- $E_2$ = -182 MPas

- $F_2$ = -159 MPas

- $G_2$ = 0.223 m

The following table shows IFR at $Q_D$ = 500 ml/min and different blood flow rates $Q_B$.

| $Q_B$ [ml/min] | 200 | 300 | 400 | 500 |
|---|---|---|---|---|
| IFR [ml/min] | 15.4 | 23.1 | 30.7 | 38.4 |

Figure 2 shows a comparison of the internal flow rates determined according to the method of the present disclosure and experimentally determined internal flow rates taken from D. Schneditz et al.: "Internal filtration, filtration fraction, and blood flow resistance in high- and low-flux dialyzers", Clin. Hemorheol. Microcirc. 58 (2014) 455-469.

**Example 2**

Internal filtration rates within a hemodialyzer (Theranova® 400, Gambro Dialysatoren GmbH, 72379 Hechingen, Germany) were determined according to one embodiment of the method of the present disclosure, and compared to internal flow rates determined experimentally (from A. Lorenzin et al.: "Quantification of Internal Filtration in Hollow Fiber Hemodialyzers with Medium Cut-off Membrane", Blood Purif. 46 (2018) 196-204).

The following physical properties of the hemodialyzer were used for the determination:

- hollow fiber inner diameter $d_B$ = 180 $\mu$m
- Membrane wall thickness $\delta_M$ = 35 $\mu$m
- Membrane porosity $\varepsilon_M$ = 0.5
- Number of fibers N = 13,000
- Effective length L = 236 mm
- Ultrafiltration coefficient $K_{UF}$ = 48 ml/(h*mm Hg)
- half distance between adjacent fibers $\delta_\varepsilon$ = 41.9 $\mu$m
- Hemodialyzer housing inner diameter $d_H$ = 38 mm

Values for blood viscosity of $\mu_B$ = 5.0 mPas and dialysate viscosity of $\mu_D$ = 0.96 mPas were used.

Internal filtration rate IFR was determined for a dialysis flow rate $Q_D$ of 500 ml/min, and blood flow rates $Q_B$ of 300 ml/min and 400 ml/min, respectively.

The values for the following constant parameters were obtained as described above.

- K = 0.116 $\mu$m$^2$s/kg

- $R_1$ = 1.24

- $R_2$ = 1.04

- $E_1$ = -335 MPas

- $E_2$ = -237 MPas

- $F_2$ = -206 MPas

- $G_2$ = 0.182 m

The following table shows IFR at $Q_D$ = 500 ml/min and different blood flow rates $Q_B$.

| $Q_B$ [ml/min] | 300 | 400 |
|---|---|---|
| IFR [ml/min] | 26.7 | 41.6 |

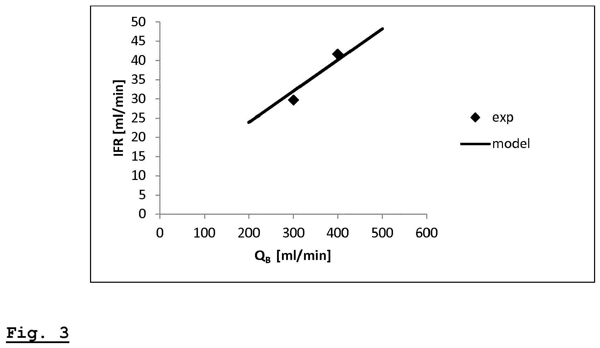

Figure 3 shows a comparison of the internal flow rates determined according to the method of the present disclosure and experimentally determined internal flow rates taken from A. Lorenzin et al.: "Quantification of Internal Filtration in Hollow Fiber Hemodialyzers with Medium Cut-off Membrane", Blood Purif. 46 (2018) 196-204.

List of reference signs

| 10 | hemodialyzer |
| --- | --- |
| 20 | bundle of hollow fiber membranes |
| 21 | hollow fiber membrane |
| 22 | membrane wall |
| L | length of hollow fiber membrane |
| $\delta_M$ | thickness of membrane wall |
| $d_B$ | internal diameter of hollow fiber membrane |
| $R_k$ | radius of space occupied by hollow fiber membrane |

**Claims**

**1.** A computer-implemented method for determining an internal filtration rate IFR within a capillary hemodialyzer (10), the method comprising

    i) acquiring, using an electronic computer processor, data relating to physical properties of the hemodialyzer (10) and of hollow fiber membranes (21) present in the hemodialyzer;
    ii) acquiring, using the processor, a blood flow rate $Q_B$ and a dialysis flow rate $Q_D$ through the hemodialyzer (10) during operation of the hemodialyzer (10);
    iii) determining, using the processor, the internal filtration rate IFR of the hemodialyzer (10), based on the data acquired in steps i and ii.

**2.** The method of claim 1, additionally comprising

    iv) acquiring, using the processor, data on a duration TD of the operation of the hemodialyzer (10) ;
    v) determining, using the processor, a total volume $V_{tot}$ of fluid exchanged through the wall (22) of the hollow fiber membranes (21) present in the hemodialyzer (10) during operation of the hemodialyzer (10), based on the internal filtration rate IFR and the data acquired in step iv.

**3.** The method of claim 1 or 2, wherein data relating to physical properties of the hemodialyzer (10) and of hollow fiber membranes (21) present in the hemodialyzer (10) is acquired from a database in operative association with the processor.

**4.** The method of any one of claims 1 to 3, wherein data relating to physical properties of the hemodialyzer (10) and of hollow fiber membranes (21) present in the hemodialyzer (10) and/or the blood flow rate and the dialysis flow rate through the hemodialyzer (10) during operation of the hemodialyzer (10) is acquired from an input device in operative association with the processor.

**5.** The method of claim 4, wherein the input device comprises a contactless reader.

**6.** The method of claim 4 or 5, wherein the input device comprises at least one user interface.

**7.** The method of any one of claims 4 to 6, wherein the input device comprises an extracorporeal blood treatment apparatus.

**8.** The method of any one of claims 1 to 7, wherein the data relating to physical properties of the hemodialyzer (10) and of hollow fiber membranes (21) present in the hemodialyzer (10) comprise a diameter $d_H$ of the housing of the hemodialyzer, a number N of the hollow fiber membranes (21) present in the hemodialyzer (10), an effective length L of the hollow fiber membranes (21) present in the hemodialyzer (10), a total surface area $A_{tot}$ of the hollow fiber membranes (21) present in the hemodialyzer (10), an internal diameter $d_B$ of the hollow fiber membranes (21) present in the hemodialyzer (10), a wall thickness $\delta_M$ of the hollow fiber membranes (21) present in the hemodialyzer (10), a porosity $\varepsilon_M$ of the hollow fiber membranes (21) present in the hemodialyzer (10), and an ultrafiltration coefficient $K_{UF}$ of the hollow fiber membranes (21) present in the hemodialyzer (10).

**8.** The method of claims 1 to 7, wherein the internal filtration rate IFR is determined according to

$$IFR = N\pi d_B \int_0^{x_i} J_v(z)\,dz$$

with

N number of hollow fiber membranes (21) present in the hemodialyzer (10);
$d_B$ internal diameter of the hollow fiber membranes (21) present in the hemodialyzer (10);
$J_v(z)$ ultrafiltration flux through the membrane wall of the hollow fiber membranes (21) present in the hemodialyzer (10);
$x_i$ location of inversion point ($J_v(x_i) = 0$) .

**9.** The method of claims 1 to 7, wherein the internal filtration rate IFR is determined according to

$$IFR = N\pi d_B K \left\{ \left(P_{B,out} - P_{D,out} - \pi_0\right)x_i \right.$$

$$- \frac{128\mu_B}{N\pi d_B^4}\left[Q_B\left(\frac{x_i^2}{2} - Lx_i\right)\right.$$

$$- N\pi d_B\left[\frac{v_2 - v_1}{6L}\left(\frac{x_i^4}{4} - L^3 x_i\right) + \frac{v_1}{2}\left(\frac{x_i^3}{3} - L^2 x_i\right)\right]\right]$$

$$\left. + \frac{\mu_D}{2N\pi f_{R_o,R_k}}\left[Q_D\frac{x_i^2}{2} - N\pi d_B\left[\frac{v_2 - v_1}{2L}\left(\frac{x_i^4}{12} - \frac{L^2}{2}x_i^2\right) + v_1\left(\frac{x_i^3}{6} - \frac{L}{2}x_i^2\right)\right]\right]\right\}$$

with

N number of hollow fiber membranes (21) present in the hemodialyzer (10);
$d_B$ internal diameter of hollow fiber membranes (21) present in the hemodialyzer (10);

K $\dfrac{K_{UF}}{A_{tot}\,\varepsilon_M}$,

$K_{UF}$ ultrafiltration coefficient of the hollow fiber membranes (21) present in the hemodialyzer (10),
$A_{tot}$ total membrane area of the hollow fiber membranes (21) present in the hemodialyzer (10) ($A_{tot} = N\pi d_B L$),
$\varepsilon_M$ membrane porosity of the hollow fiber membranes (21) present in the hemodialyzer (10);

$P_{B,out}$ blood pressure at hemodialyzer exit;
$P_{D,out}$ dialysate pressure at hemodialyzer exit;
$\Pi_0$ average oncotic pressure;
$x_i$ location of inversion point ($J_v(x_i) = 0$);
$\mu_B$ blood viscosity;
$Q_B$ blood flow rate;
L effective length of hollow fiber membranes (21) present in the hemodialyzer (10);
$v_1$ $J_v(0)$;
$v_2$ $J_v(L)$;
$\mu_D$ dialysate viscosity;
$Q_D$ dialysate flow rate;

$$f_{R_o,R_k} \quad \frac{R_k^4 - R_o^4}{16} + \frac{R_k^2}{2}\left(\frac{R_k^2}{2}\ln\left(\frac{R_o}{R_k}\right) + \frac{R_k^2 - R_o^2}{4}\right) - \frac{R_o^2}{8}(R_k^2 - R_o^2),$$

$$R_o = \frac{d_B}{2} + \delta_M,$$

$$R_k = R_o + \delta_\varepsilon,$$

$d_B$ internal diameter of the hollow fiber membranes (21) present in the hemodialyzer (10),
$\delta_M$ wall thickness of the hollow fiber membranes (21) present in the hemodialyzer (10),
$\delta_\varepsilon$ thickness of the diffusion layer around the hollow fiber membranes (21) present in the hemodialyzer (10).

**10.** The method of any one of claims 2 to 9, wherein the total volume $V_{tot}$ of fluid exchanged through the wall of the hollow fiber membranes (21) present in the hemodialyzer (10) during operation of the hemodialyzer (10) is determined according to

$$V_{tot} = \int\limits_0^{TD} IFR(t)\,dt$$

**11.** A system comprising

a) a database comprising data relating to physical properties of a plurality of capillary hemodialyzers (10) and of hollow fiber membranes (21) present therein; and/or
b) an input device configured for providing data relating to physical properties of a capillary hemodialyzer (10) and of hollow fiber membranes (21) present in the hemodialyzer (10) and/or for providing a blood flow rate and a dialysis flow rate through the hemodialyzer (10) during operation of the hemodialyzer (10);
c) an output device configured for output of data received from a computer processor in operative association with the output device;
d) a computer processor programmed for communication with the database and/or the input device, and for communication with the output device, the processor programmed for

a. acquiring data from the database and/or the input device,
b. determining an internal filtration rate IFR within a capillary hemodialyzer (10), based on the acquired data,
c. optionally, determining a total volume $V_{tot}$ of fluid exchanged through the wall of the hollow fiber membranes (21) present in the hemodialyzer (10) during operation of the hemodialyzer (10);
d. transmitting the determined internal filtration rate IFR, and, optionally, the total volume $V_{tot}$ of fluid exchanged to the output device.

**12.** The system of claim 11, wherein the input device, the output device, and the processor are contained in a mobile communication device.

**13.** The system of claim 11 or 12, wherein the input device is a graphical use interface (GUI) of a mobile communication device.

**14.** The system of any one of claims 11 to 13, wherein the output device is a display device.

**15.** A computer program for instructing a computer processor to perform the method of

a. acquiring data from a database and/or an input device in operative association with the processor;
b. determining an internal filtration rate IFR within a capillary hemodialyzer (10), based on the acquired data;
c. optionally, determining a total volume $V_{tot}$ of fluid exchanged through the wall of the hollow fiber membranes (21) present in the hemodialyzer (10) during operation of the hemodialyzer (10);

d. transmitting the determined internal filtration rate IFR, and, optionally, the total volume $V_{tot}$ of fluid exchanged to an output device in operative association with the processor.

**Fig. 1**

**Fig. 2**

Fig. 3

Europäisches
Patentamt

European
Patent Office

Office européen
des brevets

**EUROPEAN SEARCH REPORT**

Application Number

EP 19 20 2905

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEGALLAIS C ET AL: "A theoretical model to predict the in vitro performance of hemodiafilters", JOURNAL OF MEMBRANE SCIENCE, vol. 168, no. 1-2, 15 April 2000 (2000-04-15), pages 3-15, XP055683643, NL ISSN: 0376-7388, DOI: 10.1016/S0376-7388(99)00297-5 | 1-14,16 | INV. G16H20/17 A61M1/16 |
| Y | * abstract * * p.4 col.1 par.1, p.4 col.2 par.2, p.5 section over col.1-2, p.6 col.1 par.1, p.8 col.1 par.1, p.8-9 section 3.3 "Integration procedure", p.9 col.1 par.2, p.14; figures 1, 3; table 1 * | 5,7 | |
| X | DONATO D ET AL: "Optimization of dialyzer design to maximize solute removal with a two-dimensional transport model", JOURNAL OF MEMBRANE SCIENCE, ELSEVIER BV, NL, vol. 541, 11 July 2017 (2017-07-11), pages 519-528, XP085187080, ISSN: 0376-7388, DOI: 10.1016/J.MEMSCI.2017.07.018 | 1-16 | TECHNICAL FIELDS SEARCHED (IPC) G16H A61M |
| Y | * abstract * * p.520 box "Nomenclature", par. over pp.522-523, p.523 col.1 par.2 section "Computational methods", p.523 col.2 par.2; figures 1, 2, 5a; table 2a * | 5,7 | |

-/--

The present search report has been drawn up for all claims

| Place of search | Date of completion of the search | Examiner |
|---|---|---|
| Munich | 23 April 2020 | Werner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 19 20 2905

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RAFF M ET AL: "Advanced modeling of highflux hemodialysis", JOURNAL OF MEMBRANE SCIENCE, ELSEVIER BV, NL, vol. 216, no. 1-2, 1 May 2003 (2003-05-01), pages 1-11, XP004422225, ISSN: 0376-7388, DOI: 10.1016/S0376-7388(02)00552-5 | 1-16 | |
| Y | * abstract * * p.2 "Nomenclature", p.3 section "Theory"; figures 1, 7-8; table 4 * | 5,7 | |
| X | LEE J C ET AL: "Mathematical analysis for internal filtration of convection-enhanced high-flux hemodialyzer", COMPUTER METHODS AND PROGRAMS IN BIOMEDICINE, ELSEVIER, AMSTERDAM, NL; US NATIONAL LIBRARY OF MEDICINE (NLM), BETHESDA, MD, US, vol. 108, no. 1, 1 October 2012 (2012-10-01), pages 68-79, XP002746544, ISSN: 0169-2607, DOI: 10.1016/J.CMPB.2012.01.001 [retrieved on 2012-02-10] | 1-16 | |
| Y | * abstract * * p.69 col.2 par.4-p.73 col.1 par.1, Appendix B: "Mathematical model descriptions"; figures 1-2 * | 5,7 | |

-----

-----

-/--

TECHNICAL FIELDS
SEARCHED      (IPC)

The present search report has been drawn up for all claims

| Place of search | Date of completion of the search | Examiner |
|---|---|---|
| Munich | 23 April 2020 | Werner, Andreas |

CATEGORY OF CITED DOCUMENTS

X : particularly relevant if taken alone
Y : particularly relevant if combined with another
　　document of the same category
A : technological background
O : non-written disclosure
P : intermediate document

T : theory or principle underlying the invention
E : earlier patent document, but published on, or
　　after the filing date
D : document cited in the application
L : document cited for other reasons

& : member of the same patent family, corresponding
　　document

EPO FORM 1503 03.82 (P04C01)

Europäisches
Patentamt
European
Patent Office
Office européen
des brevets

# EUROPEAN SEARCH REPORT

Application Number

EP 19 20 2905

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SAKIYAMA R ET AL: "Effect of blood flow rate on internal filtration in a high-flux dialyzer with polysulfone membrane", JOURNAL OF ARTIFICIAL ORGANS ; THE OFFICIAL JOURNAL OF THE JAPANESE SOCIETY FOR ARTIFICIAL ORGANS, SPRINGER-VERLAG, TO, vol. 15, no. 3, 26 April 2012 (2012-04-26) , pages 266-271, XP035109884, ISSN: 1619-0904, DOI: 10.1007/S10047-012-0643-7 * abstract * * paragraph over pp.266-267, p.267 col.2 par.2; figures 1, 5 * | 5,7 | |
| A | WO 2018/188973 A1 (GAMBRO LUNDIA AB [SE]) 18 October 2018 (2018-10-18) * p.7 l.16-34, p.12 l.26-p.13 l.26, p.15 l.3-7, p.18 l.27-p.25 l.15 * | 1-15 | |
| A | US 2014/305869 A1 (WOLFF HENRIK [DE] ET AL) 16 October 2014 (2014-10-16) * paragraph [0032] * * paragraph [0050] - paragraph [0057] * * paragraph [0033]; figure 2 * | 1-15 | TECHNICAL FIELDS SEARCHED (IPC) |
| A | JP 2005 131123 A (ASAHI KASEI MEDICAL CO LTD) 26 May 2005 (2005-05-26) * paragraph [0003] * * paragraph [0011] * * paragraph [0047] * | 1-15 | |

The present search report has been drawn up for all claims

| Place of search | Date of completion of the search | Examiner |
|---|---|---|
| Munich | 23 April 2020 | Werner, Andreas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 20 2905

This annex lists the patent family members relating to the patent documents cited in the above-mentioned European search report.
The members are as contained in the European Patent Office EDP file on
The European Patent Office is in no way liable for these particulars which are merely given for the purpose of information.

23-04-2020

| Patent document cited in search report | | Publication date | Patent family member(s) | | Publication date |
|---|---|---|---|---|---|
| WO 2018188973 | A1 | 18-10-2018 | CN | 110573237 A | 13-12-2019 |
| | | | EP | 3388139 A1 | 17-10-2018 |
| | | | WO | 2018188973 A1 | 18-10-2018 |
| US 2014305869 | A1 | 16-10-2014 | CN | 104107467 A | 22-10-2014 |
| | | | CN | 204261114 U | 15-04-2015 |
| | | | DE | 102013103816 A1 | 13-11-2014 |
| | | | EP | 2792378 A1 | 22-10-2014 |
| | | | US | 2014305869 A1 | 16-10-2014 |
| JP 2005131123 | A | 26-05-2005 | JP | 4190394 B2 | 03-12-2008 |
| | | | JP | 2005131123 A | 26-05-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 3102312 B1
- EP 3102314 B1

### Non-patent literature cited in the description

- **A. LORENZIN et al.** Quantification of internal filtration in hollow fiber hemodialyzers with Medium cut-off membrane. *Blood Purif.,* 2018, vol. 46, 196-204
- Polyflux® 210H. Gambro Dialysatoren GmbH
- **D. SCHNEDITZ et al.** Internal filtration, filtration fraction, and blood flow resistance in high- and low-flux dialyzers. *Clin. Hemorheol. Microcirc.,* 2014, vol. 58, 455-469
- Theranova® 400. Gambro Dialysatoren GmbH
- **A. LORENZIN et al.** Quantification of Internal Filtration in Hollow Fiber Hemodialyzers with Medium Cut-off Membrane. *Blood Purif.,* 2018, vol. 46, 196-204
- **LORENZIN et al.** Quantification of Internal Filtration in Hollow Fiber Hemodialyzers with Medium Cut-off Membrane. *Blood Purif.,* 2018, vol. 46, 196-204